# EUROPEAN PATENT SPECIFICATION

(11) **EP 3 260 039 B1**
(45) Date of publication and mention of the grant of the patent: **01.01.2020**
(21) Application number: 17157111.0
(22) Date of filing: 21.02.2017
(51) Int. Cl.: A61B 1/32, A61B 1/31, A61B 1/303, A61B 1/00, A61B 1/06, A61B 1/07, A61B 17/02

(54) **DISPOSABLE MEDICAL DEVICE WITH A LIGHTING EFFECT**
WEGWERFBARE MEDIZINISCHE VORRICHTUNG MIT EINER BELEUCHTUNGSEINHEIT
DISPOSITIF MÉDICAL JETABLE AVEC UN EFFET D'ÉCLAIRAGE

(43) Date of publication of application: 27.12.2017
(73) Proprietor: Tsai, Yih-Chiou, Taichung (TW)
(72) Inventor: Tsai, Yih-Chiou, Taichung (TW)
(74) Representative: Viering, Jentschura & Partner mbB Patent- und Rechtsanwälte

(56) References cited:
- WO-A1-2012/010857
- CN-U- 203 841 677
- US-A- 4 300 541
- US-A- 5 165 387
- US-A1- 2009 198 108
- US-A1- 2014 275 790

## Description

### 1. Field of Invention

The present invention relates to a disposable medical device, and more particularly to a disposable medical device that may provide an auxiliary lighting effect in visual inspection or treatment, may deposit a light assembly on the disposable medical device depending on need, and may reduce waste of resources and solve the environmental problems effectively.

### 2. Description of the Related Art

Conventional disposable medical devices include anal speculums, vaginal speculums, endoscopes or proctoscopes, etc. In the screening or treatment, the conventional disposable medical device is inserted into the patient's examination organs (such as the anus or vagina, etc.), and an appropriate light source is needed for clearly observing the status of the screening or treatment sites. A conventional folding searchlight is used to provide a lighting effect, may be partially shielded by the medical personnel in use, and cannot be completely irradiated into the internal organs of a human. In addition, the conventional folding searchlight is expensive and heavy, and a plug-in power supply is needed for the conventional folding searchlight. When patients are at remote areas far away from the medical institution, this will cause a lot of inconvenience, and the requirement of an external power supply may make the device not always readily available for use.

In view of the above-described problems, a conventional disposable medical device with a lighting effect has been designed and manufactured. Though the conventional disposable medical device may provide an auxiliary lighting effect to the user, a light assembly is directly and securely deposited on the conventional disposable medical device, the structure of the conventional disposable medical device may be complicated, and the assembly time and the manufacturing cost are increased. Additionally, whether the light assembly is needed or not for the visual screening or treatment, in either case the entire disposable medical device is required to be discarded after use under the safety and hygiene considerations, and this may cause unnecessary waste of resources and other related environmental issues. Document US 2009/198108 A1 discloses a disposable medical device with a detachable light source in a distally sealed compartment to prevent contamination.

The disposable medical device with a lighting effect in accordance with the present invention mitigates or obviates the aforementioned problems.

The primary objective of the present invention is to provide a disposable medical device that may provide an auxiliary lighting effect in visual inspection or treatment, may deposit a light assembly on the disposable medical device according to a user's need conveniently, and may reduce waste of resources and solve the environmental problems effectively.

The disposable medical device with a lighting effect in accordance with the present invention has a body and a light assembly. The body has an inspection portion, a grip portion, and a placement portion. The light assembly is detachably connected to the body and has an outer casing, a lighting module, and a power supply module. The outer casing is detachably connected to the placement portion of the body, and has a chamber, a communicating hole, and a through hole. The lighting module is deposited in the outer casing and has a luminous body and a pressing-conductive arm. The pressing-conductive arm is swingably deposited in the outer casing, is selectively and electrically connected to the luminous body, and has an abutting portion and a conductive portion. The power supply module is deposited in the chamber, and is selectively and electrically connected to the lighting module and the conductive portion of the pressing-conductive arm.

Other objectives, advantages and novel features of the invention will become more apparent from the following detailed description when taken in conjunction with the accompanying drawings.

### IN THE DRAWINGS:

Fig. 1 is a perspective view of a first embodiment of a disposable medical device with a lighting effect in accordance with the present invention;
Fig. 2 is an exploded perspective view of the disposable medical device in Fig. 1;
Fig. 3 is a side view in partial section of the disposable medical device in Fig. 1;
Fig. 4 is an enlarged perspective view of a light assembly of the disposable medical device in Fig. 1;
Fig. 5 is an exploded perspective view of the light assembly of the disposable medical device in Fig. 4;
Fig. 6 is a top view of the light assembly of the disposable medical device in Fig. 4 under an unfolded condition;
Fig. 7 is an enlarged and operational side view in partial section of the disposable medical device in Fig. 1;
Fig. 8 is a perspective view of a second embodiment of a disposable medical device with a lighting effect in accordance with the present invention;
Fig. 9 is an exploded perspective view of the disposable medical device in Fig. 8;
Fig. 10 is an enlarged and operational side view in partial section of the disposable medical device in Fig. 8;
Fig. 11 is a perspective view of a third embodiment of a disposable medical device with a lighting effect in accordance with the present invention;
Fig. 12 is an exploded perspective view of the disposable medical device in Fig. 11; and
Fig. 13 is an enlarged and operational side view in partial section of the disposable medical device in Fig. 11.

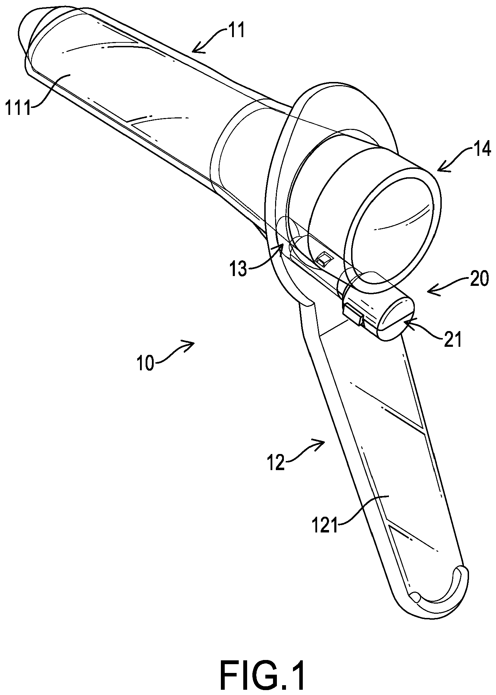

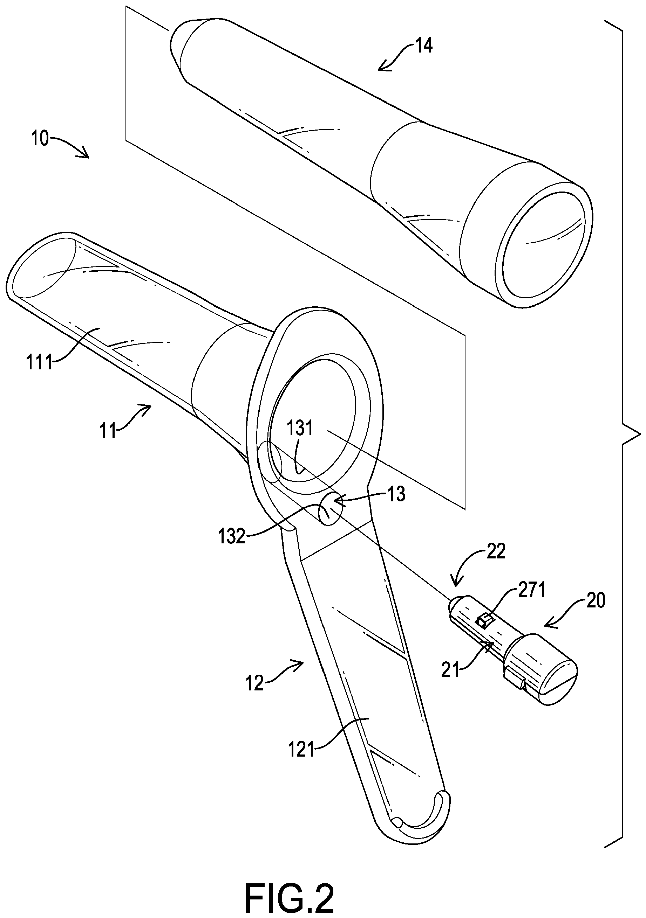

With reference to Figs. 1 to 3, a disposable medical device with a lighting effect not part of the present invention has a body 10 and a light assembly 20.

The body 10 has an inspection portion 11, a grip portion 12, and a placement portion 13. The inspection portion 11 is used for intrusive screening or treatment of a patient's organs. The grip portion 12 is connected to the inspection portion 11 for gripping by the medical personnel such as doctors. The placement portion 13 is deposited on the body 10 and toward the inspection portion 11.

The body 10 is an anal speculum, the inspection portion 11 of the body 10 is a transparent inspection tube 111, and the grip portion 12 is a grab handle 121 connected to the inspection tube 111. The placement portion 13 is a positioning recess 131 with an opening 132 and is formed between the inspection tube 111 and the grab handle 121. Furthermore, the inspection tube 111, the grab handle 121, and the positioning recess 131 are formed as a single piece by injection molding. Additionally, the body 10 has a pushing tube 14 corresponding to and deposited in the inspection tube 111.

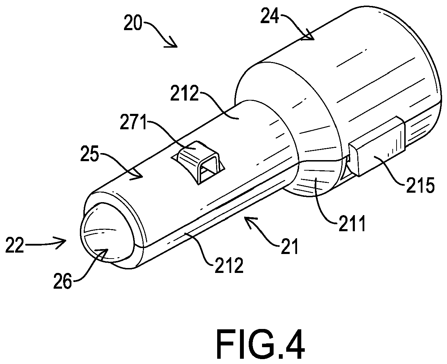

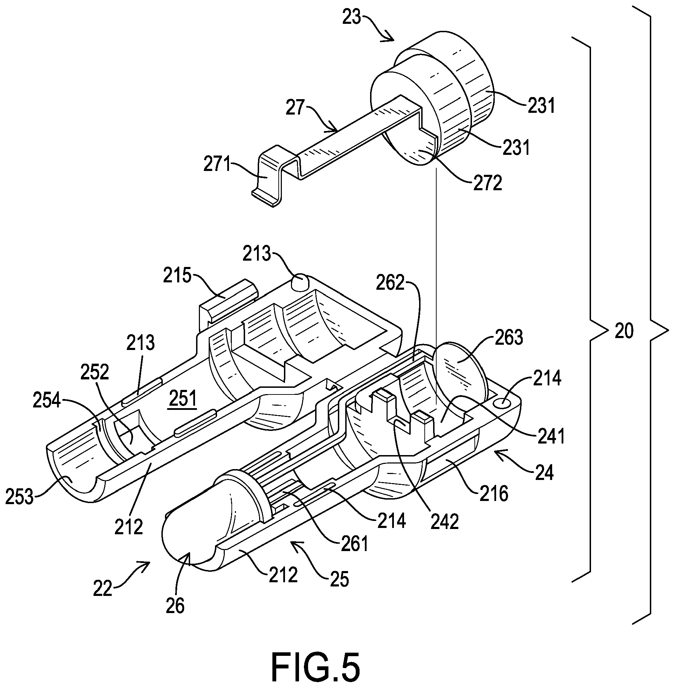

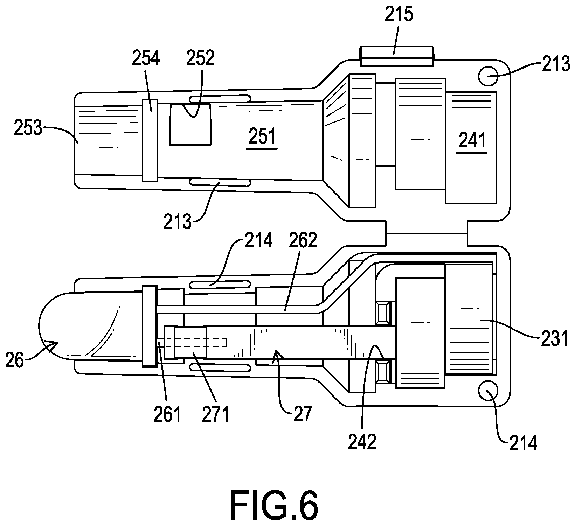

With reference to Figs. 4 to 6, the light assembly 20 is detachably connected to the body 10 and has an outer casing 21, a lighting module 22, and a power supply module 23. The outer casing 21 is a hollow shell made by plastic molding, is detachably connected to the placement portion 13 of the body 10, and has a front end extending in the positioning recess 131 of the body 10 via the opening 132 of the placement portion 13. The outer casing 21 has a base 24 and an extending seat 25. The base 24 has a chamber 241 for accommodating the power supply module 23. Furthermore, the base 24 has a through recess 242 formed at a front end of the base 24 and communicating with the chamber 241 of the base 24.

The extending seat 25 is connected to the front end of the base 24 and has an outer diameter smaller than an outer diameter of the base 24, and the outer casing 21 has two different diameters to form a stepped surface 211 between the base 24 and the extending seat 25. The extending seat 25 has a mounting recess 251, a through hole 252, and a communicating hole 253. The mounting recess 251 is formed in the extending seat 25 adjacent to the base 24 to communicate with the chamber 241 of the base 24 via the through recess 242. The through hole 252 is formed through an external surface of the extending seat 25 and communicates with the mounting recess 251. The communicating hole 253 is formed through a front end of the extending seat 25 that is opposite the base 24, and the communicating hole 253 communicates with the mounting recess 251. Additionally, the extending seat 25 has an engaging recess 254 annularly formed in an inner surface of the extending seat 25 between the through hole 252 and the communicating hole 253.

Furthermore, the base 24 and the extending seat 25 are connected to each other by injection molding, and the base 24 and the extending seat 25 are composed by two half-casings 212 that may cover each other. Each one of the half-casings 212 has a connecting side and a locking side. The two connecting sides of the two half-casings 212 are connected to each other to enable the two locking sides of the two half-casings 212 to move toward or away from each other. The outer casing 21 has multiple engaging protrusions 213 and multiple engaging holes 214 deposited on the two half-casings 212 adjacent to the two locking sides of the two half-casings 212 to enable the two half-casings 212 to form a closed structure by the engaging protrusions 213 engaging with the engaging holes 214.

Additionally, each one of the engaging protrusions 213 may be convex or elongated in shape, and each one of the engaging holes 214 has a shape corresponding to the shape of a corresponding engaging protrusion 213. Further, the outer casing 21 has a locking tab 215 and a locking recess 216 respectively deposited on the two half-casings 212 adjacent to the base 24 to enable the two half-casing 212 to connect with each other by an engagement between the locking tab 215 and the locking recess 216, and this may improve the connecting structural strength between the two half-casings 212 to prevent the two half-casings 212 from separating in use.

The lighting module 22 is deposited in the outer casing 21 and has a luminous body 26 and a pressing-conductive arm 27. The luminous body 26 is deposited in the engaging recess 254 of the extending seat 25 and has a front end extending out of the front end of the extending seat 25 via the communicating hole 253, and this enables the front end of the luminous body 26 to extend in the positioning recess 131 of the body 10 and to face the inspection tube 111. Further, the luminous body 26 may be a light-emitting diode (LED), and has a first conductive wire 261, a second conductive wire 262, and an electrode sheet 263. The first conductive wire 261 is electrically connected to the luminous body 26 and is deposited in the mounting recess 251 of the extending seat 25. The second conductive wire 262 is electrically connected to the luminous body 26 and is deposited in the chamber 241 of the base 24 via the mounting recess 251. The electrode sheet 263 is deposited in the chamber 241 of the base 24 and is electrically connected to the second conductive wire 262.

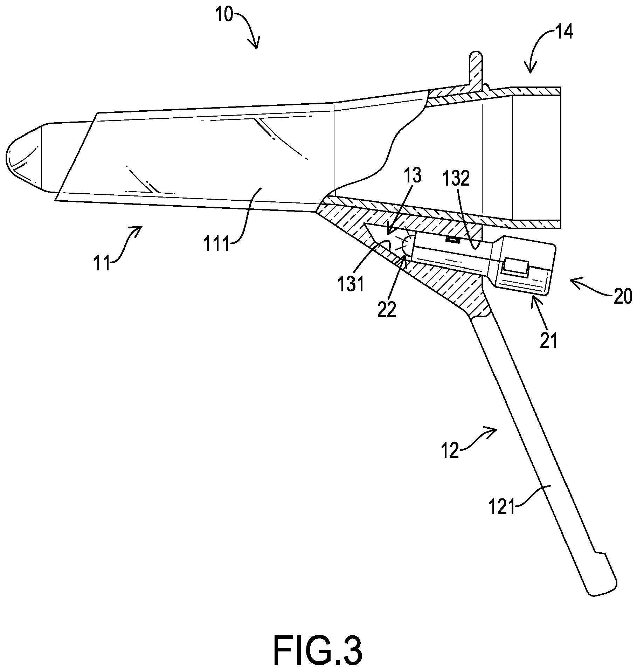

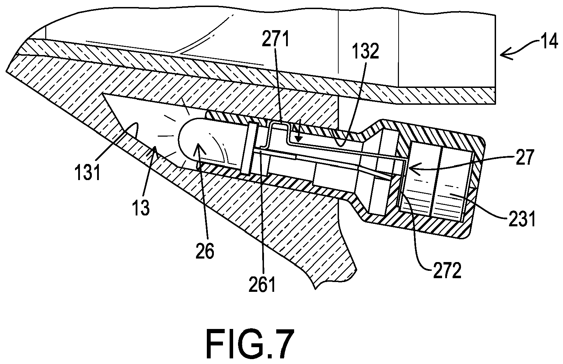

The pressing-conductive arm 27 may be an elongated metal sheet, is swingably deposited in the outer casing 21, and is selectively and electrically connected to the luminous body 26. The pressing-conductive arm 27 has an outer end, an inner end, an abutting portion 271 and a conductive portion 272. The abutting portion 271 may be a curved elastic plate, is formed on the outer end of the pressing-conductive arm 27, and extends out of the outer casing 21 via the through hole 252. The abutting portion 27 has a free end deposited adjacent to the first conductive wire 261 of the luminous body 26 at a spaced interval. With reference to Figs. 3 and 7, when the light assembly 20 is connected to the body 10 the pressing-conductive arm 27 is pressed by the body 10, and this enables the free end of the abutting portion 271 to move toward and to contact the first conductive wire 261.

With further reference to Fig. 4, when the light assembly 20 is separated from the body 10, the pressing-conductive arm 27 may be restored to the original position, and this may separate the free end of the abutting portion 271 from the first conductive wire 261. The inner end of the pressing-conductive arm 27 is opposite the outer end of the pressing-conductive arm 27, and extends in the chamber 241 of the base 24 via the mounting recess 251. The conductive portion 272 may be an electrode sheet and is deposited on the inner end of the pressing-conductive arm 27.

The power supply module 23 is deposited in the outer casing 21, is selectively and electrically connected to the lighting module 22, and has multiple mercury batteries 231 stacked with each other in the chamber 241 of the base 24. The batteries 231 are electrically connected to the luminous body 26 via the electrode sheet 263, the second conductive wire 262, the conductive portion 272 and the abutting portion 271 of the pressing-conductive arm 27, and the first conductive wire 261 when the pressing-conductive arm 27 presses against the first conductive wire 261.

When the first embodiment of the disposable medical device with a lighting effect is in use, with reference to Figs. 3, 4, and 7, an end of the light assembly 20 that has the luminous body 26 mounted thereon is pushed in the positioning recess 131 via the opening 132 of the placement portion 13. When the light assembly 20 is moved relative to the body 10, the abutting portion 271 of the pressing-conductive arm 27 that extends out of the outer casing 21 via the through hole 252 may move toward the first conductive wire 261 when the outer casing 21 is moved relative to the positioning recess 131. When the free end of the abutting portion 271 abuts against the first conductive wire 261, an electrical conduction is formed among the first conductive wire 261 of the luminous body 26, the abutting portion 271 and the conductive portion 272 of the pressing-conductive arm 27, the batteries 231, and the second conductive wire 262. Then, the power required for the luminous body 26 to emit light can be provided by the batteries 231.

When the luminous body 26 emits the light, the medical personnel may grab the grab handle 121 and insert the inspection tube 11 and the pushing tube 14 into a patient's anus to stretch an inner wall of the patient's anus and to observe the internal status of the patient's anus. The light of the luminous body 26 may provide an auxiliary lighting effect to the medical personnel during the inspection.

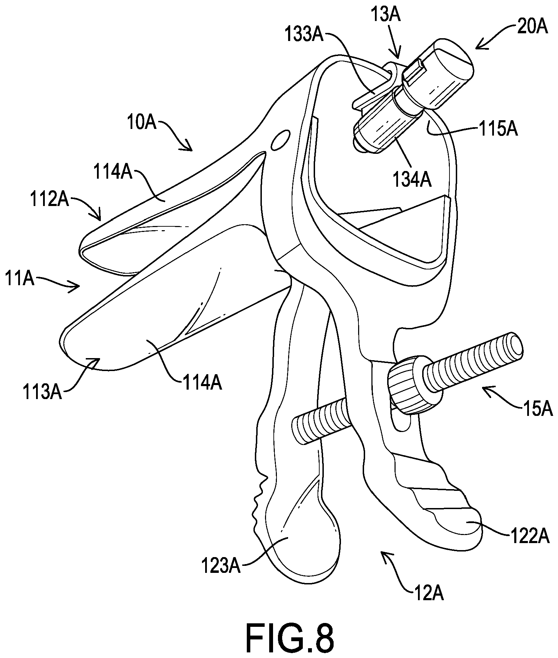

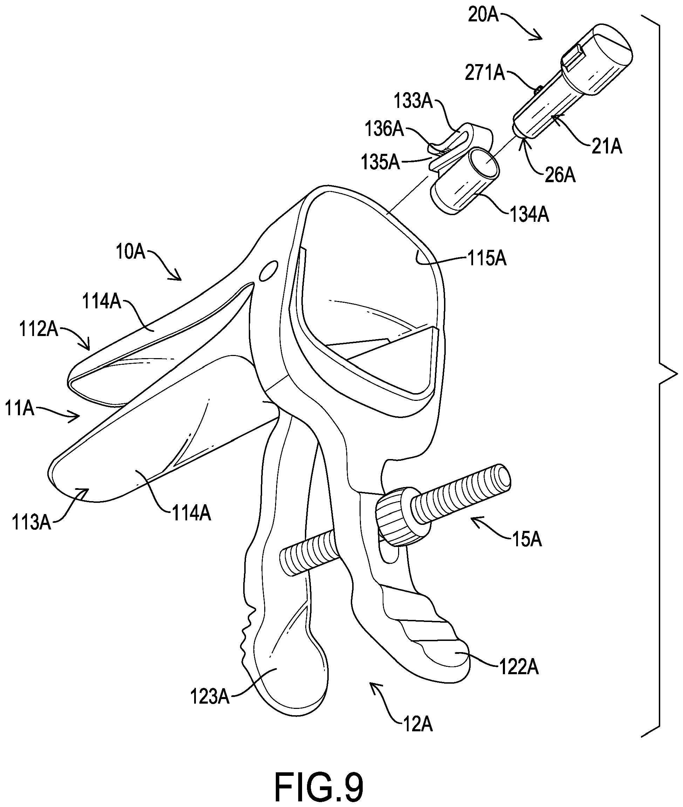

With reference to Figs. 8 to 10, a disposable medical device with a lighting effect is substantially the same as the disposable medical device as shown in Figs. 1 to 3 except for the following features. The body 10A is a vaginal speculum, and the inspection portion 11A of the body 10A has an upper jaw 112A and a lower jaw 113A. The lower jaw 113A is pivotally connected to the upper jaw 112A, and each one of the upper jaw 112A and the lower jaw 113A has a tongue segment 114A. The tongue segments 114A of the upper jaw 112A and the lower jaw 113A may move toward or away from each other to change an angle between the upper jaw 112A and the lower jaw 113A. Furthermore, the inspection portion 11A has a window 115A formed at rear ends of the upper jaw 112A and the lower jaw 113A.

The grip portion 12A has two operating stems 122A, 123A respectively connected to and controlling the movements of the upper jaw 112A and the lower jaw 113A. In addition, the body 10A has an adjusting structure 15A deposited between the operating stems 122A, 123A to drive and control the movements of the tongue segments 114A of the upper jaw 112A and the lower jaw 113A.

The placement portion 13A may be a fixture, is detachably connected to the upper jaw 112A or the lower jaw 113A to extend in the window 115A, and has a clamping body 133A and a mounting sleeve 134A. The clamping body 133A is connected to the rear end of the upper jaw 112A or the rear end of the lower jaw 113A, and has a clamping mouth 135A and multiple engaging teeth 136A. The clamping mouth 135A is a slit and has an inner surface, and the engaging teeth 136A are formed on and protrude from the inner surface of the clamping mouth 135A to increase the engaging and positioning effect between the clamping body 133A and one of the upper and lower jaws 112A, 113A.

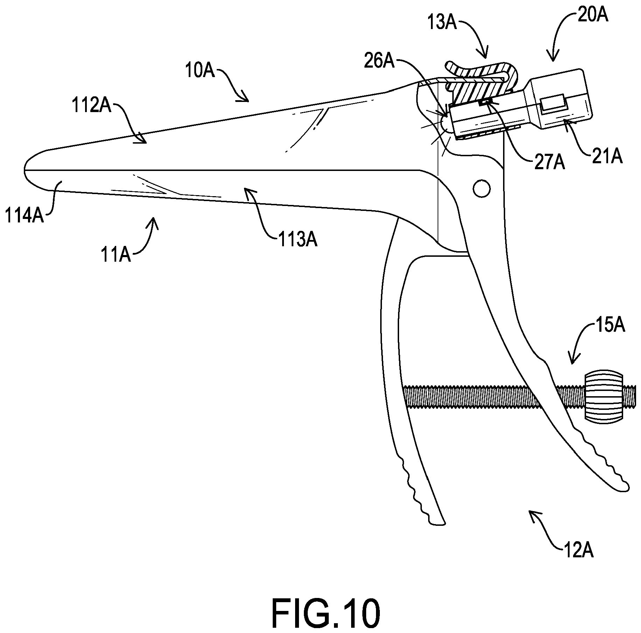

Furthermore, the clamping body 133A is connected to the rear end of the upper jaw 112A, and the mounting sleeve 134A may be a hollow tube, is connected to the clamping body 133A, and extends in the window 115A. The outer casing 21A of the light assembly 20A is mounted in the mounting sleeve 134A to make the luminous body 26A toward the inspection portion 11A. With further reference to Fig. 10, when the outer casing 21A is connected to the mounting sleeve 134A, the abutting portion 271A of the pressing-conductive arm 27A is pressed by the mounting sleeve 134A and abuts the luminous body 26A to form the electrical conduction.

When the disposable medical device with a lighting effect is in use, with reference to Figs. 8 to 10, the medical personnel may deposit the clamping body 133A on one of the upper and lower jaws 112A, 113A, and then insert the outer casing 21A of the light assembly 20A in the mounting sleeve 134A to connect the light assembly 20A with the body 10A. In addition, the medical personnel also can connect the outer casing 21A of the light assembly 20A with the mounting sleeve 134A firstly, and deposit the clamping body 133A on one of the upper and lower jaws 112A, 113A. As the outer casing 21A moves relative to the mounting sleeve 134A, the abutting portion 271A of the pressing-conductive arm 27A that extends out of the outer casing 21A may move close to and abut the luminous body 26A by the movement of the outer casing 21A relative to the mounting sleeve 134A, and this may enable the luminous body 26A to emit light.

With reference to Fig. 10, the tongue segments 114A of the upper and lower jaws 112A, 113A abut each other and are inserted into a patient's vagina, and the medical personnel may control the opening status between the tongue segments 114A of the upper and lower jaws 112A, 113A by operating the operating stems 122A, 123A to stretch an inner wall of the patient's vagina and to observe the internal status of the patient's vagina or the cervix. Additionally, the angle between the tongue segments 114A under the opening status can be held by the adjusting structure 15A, and the medical personnel may inspect with an auxiliary lighting effect that is provided by the luminous body 26A.

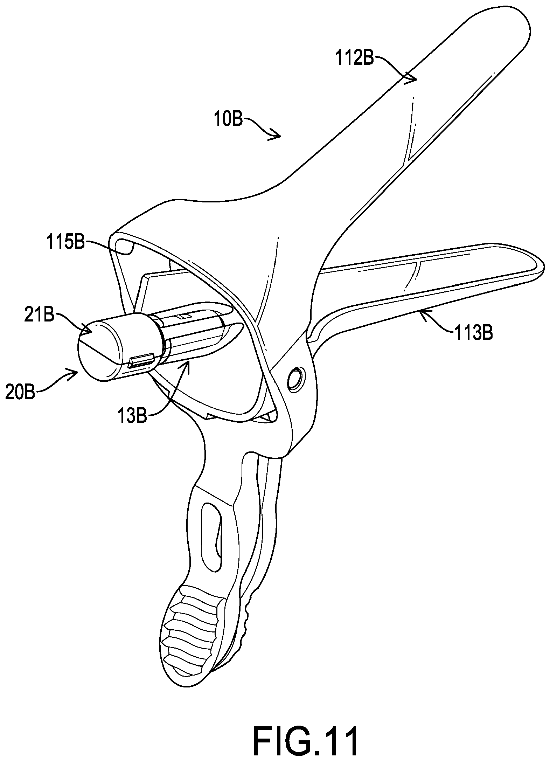

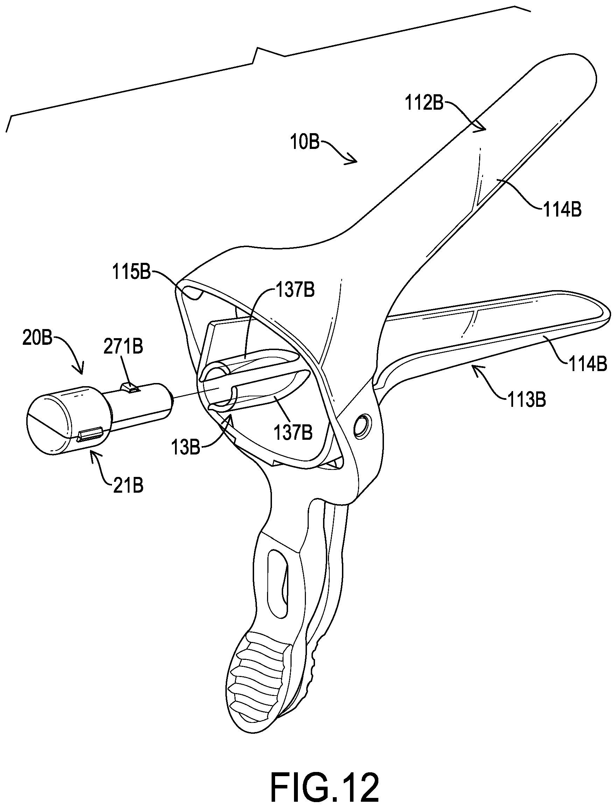

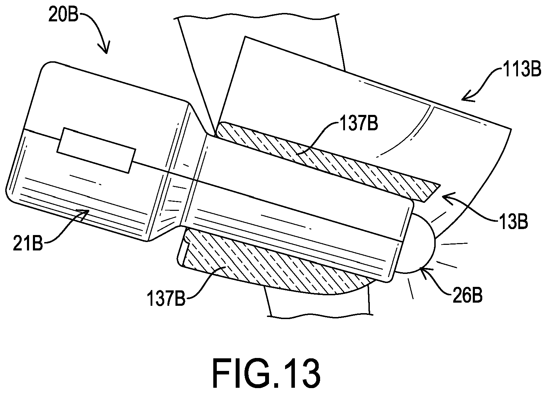

With reference to Figs. 11 to 13, a disposable medical device with a lighting effect in accordance with the present invention is substantially the same as the disposable medical device as shown in Figs. 8 to 10 except for the following features. The placement portion 13B is formed on an inner surface of one of the upper jaw 112B and the lower jaw 113B as a single piece adjacent to the rear end of said jaw 112B, 113B, and the placement portion 13B is a hollow pipe and has a front opening and a rear opening. The front opening of the placement portion 13B is deposited in a space that is formed by the upper jaw 112B and the lower jaw 113B, and the rear opening of the placement portion 13B is deposited between the upper and lower jaws 112B, 113B adjacent to the window 115B.

Further, the placement portion 13B has two curved panels 137B facing to each other at a spaced interval to form the hollow pipe, and each one of the curved panels 137B has an extending direction same as the extending directions of the tongue segments 114B of the upper and lower jaws 112B, 113B. Additionally, the placement portion 13B is formed on and protrudes from the inner surface of the lower jaw 113B as a single piece adjacent to the rear end of the lower jaw 113B, and the outer casing 21B of the light assembly 20B engages with the curved panels 137B to make the luminous body 26B toward the upper and lower jaws 112B, 113B.

When the third embodiment of the disposable medical device with a lighting effect is in use, with reference to Figs. 11 to 13, the end of the light assembly 20B that has the luminous body 26B deposited thereon is mounted between the curved panels 137B via the rear opening of the placement portion 13B. As the outer casing 21B moves relative to the curved panels 137B, the abutting portion 271B of the pressing-conductive arm 27B that extends out of the outer casing 21B may move toward and abut the luminous body 26B to enable the luminous body 26B to emit light under the electrical conduction. The upper and lower jaws 112B, 113B are inserted into the patient's vagina to stretch the inner wall of the patient's vagina and to observe the internal status of the patient's vagina or the cervix.

According to the above-mentioned structural relationships and features of the embodiments in accordance with the present invention, the through hole 252 is formed through the outer casing 21, 21A, 21B to enable the abutting portion 271, 271A, 271B of the pressing-conductive arm 27, 27A to extend out of the outer casing 21, 21A, 21B. When the light assembly 20, 20A, 20B is connected to the placement portion 13, 13A, 13B of the body 10, 10A, 10B, the abutting portion 271, 271A, 271B of the pressing-conductive arm 27, 27A is pressed to move into the outer casing 21, 21A, 21B to abut against the first conductive wire 261 of the luminous body 26, 26A, 26B. Then, the electrical conduction is formed among the first conductive wire 261 of the luminous body 26, 26A, 26B, the abutting portion 271, 271A, 271B and the conductive portion 272 of the pressing-conductive arm 27, 27A, the batteries 231, and the second conductive wire 262. Then, the power required for the luminous body 26, 26A, 26B to emit light can be provided by the batteries 231.

Furthermore, the light assembly 20, 20A, 20B is detachably connected to the body 10, 10A, 10B, and this makes the light assembly 20, 20A, 20B applicable to disposable medical devices of different types or sizes, such as anal speculums or vaginal speculums, by directly depositing the light assembly 20, 20A, 20B on the disposable medical devices conveniently and easily. Then, the medical personnel may deposit the light assembly 20, 20A, 20B on the body 10, 10A, 10B of the disposable medical device depending on need to provide an auxiliary lighting effect in visual inspection or treatment, and may reduce waste of resources and solve the environmental problems effectively.

Even though numerous characteristics and advantages of the present invention have been set forth in the foregoing description, together with details of the structure and function of the invention, the disclosure is illustrative only, and changes may be made in detail, especially in matters of shape, size, and arrangement of parts within the principles of the invention to the full extent indicated by the broad general meaning of the terms in which the appended claims are expressed.

## Claims

1. A disposable medical device with a lighting effect, the disposable medical device comprising:
a body (10, 10A, 10B) having
an inspection portion (11, 11A);
a grip portion (12, 12A) connected to the inspection portion (11, 11A); and
a placement portion (13, 13A, 13B) deposited on an inner surface of the inspection portion (11, 11A) of the body (10, 10A, 10B) as a single piece adjacent to a rear end of the inspection portion (11, 11A) facing toward the inspection portion (11, 11A), and having
a front opening;
a rear opening communicating with the front opening; and
two curved panels (137B) facing to each other at a spaced interval between the front opening and the rear opening to form a hollow pipe, and each one of the curved panels (137B) having an extending direction same as an extending direction of the inspection portion (11, 11A); and
a light assembly (20, 20A, 20B) detachably connected to the body (10, 10A, 10B) and having
an outer casing (21, 21A, 21B) detachably engaging with the curved panels (137B) of the placement portion (13, 13A, 13B) of the body (10, 10A, 10B), and having
a base (24) and an extending seat (25);
wherein the extending seat (25) has an outer diameter smaller than an outer diameter of the base (24); and the outer casing (21, 21A, 21B) has a stepped surface (211) deposited between the base (24) and the extending seat (25);
a chamber (241) formed in the base (24) of the outer casing (21, 21A, 21B);
a communicating hole (253) formed through the front end of the extending seat (25), and being opposite the chamber (241); and
a through hole (252) formed through the outer casing (21, 21A, 21B) between the chamber (241) and the communicating hole (253);
a lighting module (22) deposited in the outer casing (21, 21A, 21B) and having
a luminous body (26, 26A, 26B) deposited in the outer casing (21, 21A, 21B) and having a front end extending in the placement portion (13, 13A, 13B) via the communicating hole (253) and facing the inspection portion (11, 11A); and
a pressing-conductive arm (27, 27A) swingably deposited in the outer casing (21, 21A, 21B), selectively and electrically connected to the luminous body (26, 26A, 26B), and having
an outer end;
an inner end;
an abutting portion (271, 271A, 271B) being a curved elastic plate, formed on the outer end of the pressing-conductive arm (27, 27A), extending out of the outer casing (21, 21A, 21B) via the through hole (252), and having a free end deposited adjacent to the luminous body (26, 26A, 26B) at a spaced interval; and
a conductive portion (272) deposited on the inner end of the pressing-conductive arm (27, 27A); and
a power supply module (23) deposited in the chamber (241) of the outer casing (21, 21A, 21B), and selectively and electrically connected to the lighting module (22) and the conductive portion (272) of the pressing-conductive arm (27, 27A);
wherein the light assembly (20, 20A, 20B) is connected to the body (10, 10A, 10B), the end of the light assembly (20B) that has the luminous body (26B) deposited thereon is mounted between the curved panels (137B) via the rear opening of the placement portion (13B), the pressing-conductive arm (27, 27A) is pressed by the body (10, 10A, 10B) to enable the free end of the abutting portion (271, 271A, 271B) to move toward and to contact the luminous body (26, 26A, 26B), the power supply module (23) provides power to the luminous body (26, 26A, 26B) via the pressing-conductive arm (27, 27A).

2. The disposable medical device as claimed in claim 1, wherein
the base (24) has a through recess (242) formed at a front end of the base (24) and communicating with the chamber (241);
the extending seat (25) is connected to the front end of the base (24), and has a mounting recess (251) formed in the extending seat (25) adjacent to the base (24) and communicating with the chamber (241) via the through recess (242);
the through hole (252) is formed through the extending seat (25) and communicates with the mounting recess (251);
the communicating hole (253) is formed through a front end of the extending seat (25) that is opposite the base (24), and communicates with the mounting recess (251); and
the luminous body (26) has
a first conductive wire (261) electrically connected to the luminous body (26) and deposited in the mounting recess (251) of the extending seat (25);
a second conductive wire (262) electrically connected to the luminous body (26) and deposited in the chamber (241) of the base (24) via the mounting recess (251); and
an electrode sheet (263) deposited in the chamber (241) of the base (24) and electrically connected to the second conductive wire (262).

3. The disposable medical device as claimed in claim 2, wherein the power supply module (23) has multiple mercury batteries (231) stacked with each other in the chamber (241) of the base (24), the batteries (231) electrically connected to the luminous body (26, 26A, 26B) via the electrode sheet (263), the second conductive wire (262), the conductive portion (272) and the abutting portion (271, 271A, 271B) of the pressing-conductive arm (27, 27A), and the first conductive wire (261) when the pressing-conductive arm (27, 27A) presses against the first conductive wire (261).

4. The disposable medical device as claimed in claim 3, wherein
the base (24) and the extending seat (25) are connected to each other by injection molding, and are composed by two half-casings (212);
each one of the two half-casings (212) has
a locking side; and
a connecting side, the two connecting sides of the two half-casings (212) connected to each other to enable the two locking sides of the two half-casings (212) to move toward or away from each other; and
the outer casing (21, 21A, 21B) has multiple engaging protrusions (213) and multiple engaging holes (214) deposited on the two half-casings (212) adjacent to the two locking sides of the two half-casings (212) to enable the two half-casings (212) to form a closed structure by the engaging protrusions (213) engaging with the engaging holes (214).

5. The disposable medical device as claimed in claim 4, wherein the outer casing (21, 21A, 21B) has a locking tab (215) and a locking recess (216) respectively deposited on the two half-casings (212) adjacent to the base (24) to enable the two half-casing (212) to connect with each other by an engagement between the locking tab (215) and the locking recess (216).

6. The disposable medical device as claimed in claim 5, wherein
the extending seat (25) has an engaging recess (254) annularly formed in an inner surface of the extending seat (25) between the through hole (252) and the communicating hole (253); and
the luminous body (26) is deposited in the engaging recess (254) of the extending seat (25).

7. The disposable medical device as claimed in any one of claims 1 to 6, wherein
the body (10) is an anal speculum;
the inspection portion (11) of the body (10) is a transparent inspection tube (111); and
the grip portion (12) is a grab handle (121) and is connected to the inspection tube (111).

8. The disposable medical device as claimed in any one of claims 1 to 6, wherein
the body (10A, 10B) is a vaginal speculum;
the inspection portion (11 A) of the body (10A, 10B) has
an upper jaw (112A, 112B) having a tongue segment (114A, 114B);
a lower jaw (113A, 113B) pivotally connected to the upper jaw (112A, 112B) and having a tongue segment (114A, 114B) moveable toward or away from the tongue segment (114A, 114B) of the upper jaw (112A, 112B); and
a window (115A, 115B) formed at rear ends of the upper jaw (112A, 112B) and the lower jaw (113A, 113B); and
the grip portion (12A) has two operating stems (122A, 123A) respectively connected to and controlling the movements of the upper jaw (112A, 112B) and the lower jaw (113A, 113B).

9. The disposable medical device as claimed in claim 8, wherein
the placement portion (13B) is formed on an inner surface of one of the upper jaw (112B) and the lower jaw (113B) adjacent to the rear end of said jaw (112B, 113B);
the front opening is deposited in a space that is formed by the upper jaw (112B) and the lower jaw (113B); and
the rear opening is deposited between the upper and lower jaws (112B, 113B) adjacent to the window (115B).

## Patentansprüche

1. Eine wegwerfbare medizinische Vorrichtung mit Beleuchtungseffekt, wobei die wegwerfbare medizinische Vorrichtung umfasst:
einen Körper (10, 10A, 10B), umfassend
einen Untersuchungsabschnitt (11, 11A),
einen Griffabschnitt (12, 12A), der mit dem Untersuchungsabschnitt (11, 11A) verbunden ist, und
einen Platzierungsabschnitt (13, 13A, 13B), der an einer Innenfläche des Untersuchungsabschnitts (11, 11A) des Körpers (10, 10A, 10B) als ein einziges Stück benachbart zu einem hinteren Ende des Untersuchungsabschnitts (11, 11A) angeordnet ist, dem Untersuchungsabschnitt (11, 11A) zugewandt ist und umfasst:
eine vordere Öffnung,
eine hintere Öffnung, die mit der vorderen Öffnung kommuniziert, und
zwei gekrümmte Platten (137B), die einander in einem Abstand zwischen der vorderen Öffnung und der hinteren Öffnung zugewandt sind, um ein hohles Rohr auszubilden, und wobei jede der gekrümmten Platten (137B) eine Erstreckungsrichtung hat, die gleich wie eine Erstreckungsrichtung des Untersuchungsabschnitts (11, 11A) ist, und
eine Lichtanordnung (20, 20A, 20B), die lösbar mit dem Körper (10, 10A, 10B) verbunden ist und umfasst:
ein Außengehäuse (21, 21A, 21B), das lösbar mit den gekrümmten Platten (137B) des Platzierungsabschnitts (13, 13A, 13B) des Körpers (10, 10A, 10B) in Eingriff steht und umfasst:
eine Basis (24) und einen Erstreckungssitz (25),
wobei der Erstreckungssitz (25) einen Außendurchmesser hat, der kleiner als ein Außendurchmesser der Basis (24) ist,
und das Außengehäuse (21, 21A, 21B) eine abgestufte Fläche (211) aufweist, die zwischen der Basis (24) und dem Erstreckungssitz (25) angeordnet ist,
eine Kammer (241), die in der Basis (24) des Außengehäuses (21, 21A, 21B) ausgebildet ist,
ein Kommunikationsloch (253), das durch das vordere Ende des Erstreckungssitzes (25) ausgebildet ist und zu der Kammer (241) entgegengesetzt ist, und
ein Durchgangsloch (252), das durch das Außengehäuse (21, 21A, 21B) hindurch zwischen der Kammer (241) und dem Kommunikationsloch (253) ausgebildet ist,
ein Beleuchtungsmodul (22), das in dem Außengehäuse (21, 21A, 21B) angeordnet ist und umfasst:
einen Leuchtkörper (26, 26A, 26B), der in dem Außengehäuse (21, 21A, 21B) angeordnet ist und ein vorderes Ende hat, das sich über das Kommunikationsloch (253) in dem Platzierungsabschnitt (13, 13A, 13B) erstreckt und dem Untersuchungsabschnitt (11, 11A) zugewandt ist, und
einen drückleitfähigen Arm (27, 27A), der schwenkbar in dem Außengehäuse (21, 21A, 21B) angeordnet ist, selektiv und elektrisch mit dem Leuchtkörper (26, 26A, 26B) verbunden ist und umfasst:
ein äußeres Ende,
ein inneres Ende,
einen Angrenzungsabschnitt (271, 271A, 271B), der eine gekrümmte elastische Platte ist, am äußeren Ende des drückleitfähigen Arms (27, 27A) ausgebildet ist, sich über das Durchgangsloch (252) aus dem Außengehäuse (21, 21A, 21B) heraus erstreckt und ein freies Ende hat, das in einem Abstand benachbart zu dem Leuchtkörper (26, 26A, 26B) angeordnet ist, und
einen leitfähigen Abschnitt (272), der an dem inneren Ende des drückleitfähigen Arms (27, 27A) angeordnet ist, und
ein Stromversorgungsmodul (23), das in der Kammer (241) des Außengehäuses (21, 21A, 21B) angeordnet und selektiv und elektrisch mit dem Beleuchtungsmodul (22) und dem leitfähigen Abschnitt (272) des drückleitfähigen Arms (27, 27A) verbunden ist,
wobei die Lichtanordnung (20, 20A, 20B) mit dem Körper (10, 10A, 10B) verbunden ist, wobei das Ende der Lichtanordnung (20B), auf der der Leuchtkörper (26B) angeordnet ist, über die hintere Öffnung des Platzierungsabschnitts (13B) zwischen den gekrümmten Platten (137B) montiert ist,
der drückleitfähige Arm (27, 27A) durch den Körper (10, 10A, 10B) gedrückt wird, um zu ermöglichen, dass sich das freie Ende des Angrenzungsabschnitts (271, 271A, 271B) in Richtung zu dem Leuchtkörper (26, 26A, 26B) bewegt und diesen kontaktiert, wobei das Stromversorgungsmodul (23) den Leuchtkörper (26, 26A, 26B) über den drückleitfähigen Arm (27, 27A) mit Strom versorgt.

2. Die wegwerfbare medizinische Vorrichtung nach Anspruch 1, wobei
die Basis (24) eine Durchgangsaussparung (242) hat, die an einem vorderen Ende der Basis (24) ausgebildet ist und mit der Kammer (241) kommuniziert,
der Erstreckungssitz (25) mit dem vorderen Ende der Basis (24) verbunden ist und eine Montageaussparung (251) aufweist, die in dem Erstreckungssitz (25) benachbart zu der Basis (24) ausgebildet ist und über die Durchgangsaussparung (242) mit der Kammer (241) kommuniziert,
das Durchgangsloch (252) durch den Erstreckungssitz (25) hindurch ausgebildet ist und mit der Montageaussparung (251) kommuniziert,
das Kommunikationsloch (253) durch ein vorderes Ende des Erstreckungssitzes (25) ausgebildet ist, das zu der Basis (24) entgegengesetzt ist und mit der Montageaussparung (251) kommuniziert, und
der Leuchtkörper (26) umfasst:
einen ersten leitfähigen Draht (261), der elektrisch mit dem Leuchtkörper (26) verbunden ist und in der Montageaussparung (251) des Erstreckungssitzes (25) angeordnet ist,
einen zweiten leitfähigen Draht (262), der elektrisch mit dem Leuchtkörper (26) verbunden ist und über die Montageaussparung (251) in der Kammer (241) der Basis (24) angeordnet ist, und
ein Elektrodenblatt (263), das in der Kammer (241) der Basis (24) angeordnet und mit dem zweiten leitfähigen Draht (262) elektrisch verbunden ist.

3. Die wegwerfbare medizinische Vorrichtung nach Anspruch 2, wobei das Stromversorgungsmodul (23) mehrere Quecksilberbatterien (231) umfasst, die in der Kammer (241) der Basis (24) zusammengestapelt sind, wobei die Batterien (231) mit dem Leuchtkörper (26, 26A, 26B) über das Elektrodenblatt (263), den zweiten leitfähigen Draht (262), den leitfähigen Abschnitt (272) und den Angrenzungsabschnitt (271, 271A, 271B) des drückleitfähigen Arms (27, 27A) und den ersten leitfähigen Draht (261) elektrisch verbunden sind, wenn der drückleitfähige Arm (27, 27A) gegen den ersten leitfähigen Draht (261) drückt.

4. Die wegwerfbare medizinische Vorrichtung nach Anspruch 3, wobei
die Basis (24) und der Erstreckungssitz (25) durch Spritzgießen miteinander verbunden sind und aus zwei halben Gießkörpern (212) zusammengesetzt sind,
jeder der beiden halben Gießkörper (212) umfasst:
eine Verriegelungsseite und
eine Verbindungsseite, wobei die beiden Verbindungsseiten der beiden halben Gießkörper (212) miteinander verbunden sind, um zu ermöglichen, dass die beiden Verriegelungsseiten der beiden halben Gießkörper (212) sich in Richtung zueinander oder voneinander wegbewegen, und
das Außengehäuse (21, 21A, 21B) mehrere Eingriffsvorsprünge (213) und mehrere Eingriffslöcher (214) aufweist, die auf den beiden halben Gießkörpern (212) benachbart zu den beiden Verriegelungsseiten der beiden halben Gießkörper (212) angeordnet sind, um zu ermöglichen, dass die beiden halben Gießkörper (212) durch die Eingriffsvorsprünge (213), die mit den Eingriffslöchern (214) in Eingriff sind, eine geschlossene Struktur ausbilden.

5. Die wegwerfbare medizinische Vorrichtung nach Anspruch 4, wobei das Außengehäuse (21, 21A, 21B) eine Verriegelungslasche (215) und eine Verriegelungsaussparung (216) aufweist, die jeweils an den beiden halben Gießkörpern (212) benachbart zu der Basis (24) angeordnet sind, um zu ermöglichen, dass die beiden halben Gießkörper (212) durch einen Eingriff zwischen der Verriegelungslasche (215) und der Verriegelungsaussparung (216) miteinander verbunden werden können.

6. Die wegwerfbare medizinische Vorrichtung nach Anspruch 5, wobei
der Erstreckungssitz (25) eine Eingriffsaussparung (254) aufweist, die ringförmig in einer Innenfläche des Erstreckungssitzes (25) zwischen dem Durchgangsloch (252) und dem Kommunikationsloch (253) ausgebildet ist, und
der Leuchtkörper (26) in der Eingriffsaussparung (254) des Erstreckungssitzes (25) angeordnet ist.

7. Die wegwerfbare medizinische Vorrichtung nach einem der Ansprüche 1 bis 6,
wobei
der Körper (10) ein Analspekulum ist,
der Untersuchungsabschnitt (11) des Körpers (10) ein transparentes Untersuchungsrohr (111) ist, und
der Griffabschnitt (12) ein Haltegriff (121) ist, und mit dem Untersuchungsrohr (111) verbunden ist.

8. Die wegwerfbare medizinische Vorrichtung nach einem der Ansprüche 1 bis 6,
wobei
der Körper (10A, 10B) ein Vaginalspekulum ist,
der Untersuchungsabschnitt (11A) des Körpers (10A, 10B) umfasst:
eine obere Klemmbacke (112A, 112B) mit einem Zungensegment (114A, 114B),
eine untere Klemmbacke (113A, 113B), die schwenkbar mit der oberen Klemmbacke (112A, 112B) verbunden ist und ein Zungensegment (114A, 114B) aufweist, das in Richtung zu dem Zungensegment (114A, 114B) der oberen Klemmbacke (112A, 112B) oder von diesem weg bewegbar ist, und
ein Fenster (115A, 115B), das an den hinteren Enden der oberen Klemmbacke (112A, 112B) und der unteren Klemmbacke (113A, 113B) ausgebildet ist, und
der Griffabschnitt (12A) zwei Betätigungsstiele (122A, 123A) aufweist, die jeweils mit der oberen Klemmbacke (112A, 112B) und der unteren Klemmbacke (113A, 113B) verbunden sind und die Bewegungen dieser steuern.

9. Die wegwerfbare medizinische Vorrichtung nach Anspruch 8, wobei
der Platzierungsabschnitt (13B) an einer Innenfläche von einer der oberen Klemmbacke (112B) und der unteren Klemmbacke (113B) benachbart zu dem hinteren Ende der Klemmbacke (112B, 113B) ausgebildet ist,
die vordere Öffnung in einem Raum angeordnet ist, der durch die obere Klemmbacke (112B) und die untere Klemmbacke (113B) ausgebildet ist, und
die hintere Öffnung zwischen der oberen und der unteren Klemmbacke (112B, 113B) benachbart zu dem Fenster (115B) angeordnet ist.

## Revendications

1. Dispositif médical jetable avec un effet d'éclairage, le dispositif médical jetable comprenant :
un corps (10, 10A, 10B) ayant :
une partie d'inspection (11, 11A) ;
une partie de préhension (12, 12A) raccordée à la partie d'inspection (11, 11A) ; et
une partie de mise en place (13, 13A, 13B) déposée sur une surface interne de la partie d'inspection (11, 11A) du corps (10, 10A, 10B) d'un seul tenant adjacent à une extrémité arrière de la partie d'inspection (11, 11A) orientée vers la partie d'inspection (11, 11A), et ayant :
une ouverture avant :
une ouverture arrière communiquant avec l'ouverture avant ; et
deux panneaux incurvés (137B) se faisant face à un intervalle espacé entre l'ouverture avant et l'ouverture arrière afin de former un tuyau creux, et chacun des panneaux incurvés (137B) ayant une même direction d'extension qu'une direction d'extension de la partie d'inspection (11, 11A) ; et
un ensemble d'éclairage (20, 20A, 20B) raccordé, de manière détachable, au corps (10, 10A, 10B) et ayant :
un boîtier externe (21, 21A, 21B) se mettant en prise, de manière détachable, avec les panneaux incurvés (137B) de la partie de mise en place (13, 13A, 13B) du corps (10, 10A, 10B) et ayant :
une base (24) et un siège d'extension (25) ;
dans lequel le siège d'extension (25) a un diamètre externe inférieur à un diamètre externe de la base (24) ;
et le boîtier externe (21, 21A, 21B) a une surface étagée (211) déposée entre la base (24) et le siège d'extension (25) ;
une chambre (241) formée dans la base (24) du boîtier externe (21, 21A, 21B) ;
un trou de communication (253) formé à travers l'extrémité avant du siège d'extension (25), et étant opposé à la chambre (241) ; et
un trou débouchant (252) formé à travers le boîtier externe (21, 21A, 21B) entre la chambre (241) et le trou de communication (253) ;
un module d'éclairage (22) déposé dans le boîtier externe (21, 21A, 21B) et ayant :
un corps lumineux (26, 26A, 26B) déposé dans le boîtier externe (21, 21A, 21B) et ayant une extrémité avant s'étendant dans la partie de mise en place (13, 13A, 13B) via le trou de communication (253) et faisant face à la partie d'inspection (11, 11A) ; et
un bras conducteur par pression (27, 27A) déposé, de manière oscillante, dans le boîtier externe (21, 21A, 21B), raccordé sélectivement et électriquement au corps lumineux (26, 26A, 26B) et ayant :
une extrémité externe ;
une extrémité interne ;
une partie de butée (271, 271A, 271B) qui est une plaque élastique incurvée, formée sur l'extrémité externe du bras conducteur par pression (27, 27A) s'étendant hors du boîtier externe (21, 21A, 21B) via le trou débouchant (252) et ayant une extrémité libre déposée de manière adjacente au corps lumineux (26, 26A, 26B) à un intervalle espacé ; et
une partie conductrice (272) déposée sur l'extrémité interne du bras conducteur par pression (27, 27A) ; et
un module d'alimentation d'énergie (23) déposé dans la chambre (241) du boîtier externe (21, 21A, 21B) et raccordé sélectivement et électriquement au module d'éclairage (22) et la partie conductrice (272) du bras conducteur par pression (27, 27A) ;
dans lequel l'ensemble d'éclairage (20, 20A, 20B) est raccordé au corps (10, 10A, 10B), l'extrémité de l'ensemble d'éclairage (20B) qui a le corps lumineux (26B) déposé sur ce dernier, est montée entre les panneaux incurvés (137B) via l'ouverture arrière de la partie de mise en place (13B), le bras conducteur par pression (27, 27A) est comprimé par le corps (10, 10A, 10B) pour permettre à l'extrémité libre de la partie de butée (271, 271A, 271B) de se déplacer vers et être en contact avec le corps lumineux (26, 26A, 26B), le module d'alimentation d'énergie (23) fournit l'énergie au corps lumineux (26, 26A, 26B) via le bras conducteur par pression (27, 27A).

2. Dispositif médical jetable selon la revendication 1, dans lequel :
la base (24) a un évidement débouchant (242) formé au niveau d'une extrémité avant de la base (24) et communiquant avec la chambre (241) ;
le siège d'extension (25) est raccordé à l'extrémité avant de la base (24) et a un évidement de montage (251) formé dans le siège d'extension (25) adjacent à la base (24) et communiquant avec la chambre (241) via l'évidement débouchant (242) ;
le trou débouchant (252) est formé à travers le siège d'extension (25) et communique avec l'évidement de montage (251) ;
le trou de communication (253) est formé à travers une extrémité avant du siège d'extension (25) qui est opposé à la base (24) et communique avec l'évidement de montage (251) ; et
le corps lumineux (26) a :
un premier fil conducteur (261) raccordé électriquement au corps lumineux (26) et déposé dans l'évidement de montage (251) du siège d'extension (25) ;
un second fil conducteur (262) électriquement raccordé au corps lumineux (26) et déposé dans la chambre (241) de la base (24) via l'évidement de montage (251) ; et
une feuille d'électrode (263) déposée dans la chambre (241) de la base (24) et électriquement raccordée au second fil conducteur (262).

3. Dispositif médical jetable selon la revendication 2, dans lequel le module d'alimentation d'énergie (23) a plusieurs batteries au mercure (231) empilées les unes sur les autres dans la chambre (241) de la base (24), les batteries (231) étant électriquement raccordées au corps lumineux (26, 26A, 26B) via une feuille d'électrode (263), au second fil conducteur (262), à la partie conductrice (272) et à la partie de butée (271, 271A, 271B) du bras conducteur par pression (27, 27A) et au premier fil conducteur (261) lorsque le bras conducteur par pression (27, 27A) appuie contre le premier fil conducteur (261).

4. Dispositif médical jetable selon la revendication 3, dans lequel la base (24) et le siège d'extension (25) sont raccordés entre eux par moulage par injection, et sont composés par deux demi-boîtiers (212) ;
chacun des deux demi-boîtiers (212) a :
un côté de verrouillage ; et
un côté de raccordement, les deux côtés de raccordement des deux demi-boîtiers (212) étant raccordés entre eux pour permettre aux deux côtés de verrouillage des deux demi-boîtiers (212) de se déplacer l'un vers l'autre ou à distance l'un de l'autre ; et
le boîtier externe (21, 21A, 21B) a plusieurs saillies de mise en prise (213) et plusieurs trous de mise en prise (214) déposés sur les deux demi-boîtiers (212) adjacents aux deux côtés de verrouillage des deux demi-boîtiers (212) pour permettre aux deux demi-boîtiers (212) de former une structure fermée par les saillies de mise en prise (213) qui se mettent en prise avec les trous de mise en prise (214).

5. Dispositif médical jetable selon la revendication 4, dans lequel le boîtier externe (21, 21A, 21B) a une languette de verrouillage (215) et un évidement de verrouillage (216) respectivement déposés sur les deux demi-boîtiers (212) de manière adjacente à la base (24) pour permettre aux deux demi-boîtiers (212) de se raccorder entre eux par une mise en prise entre la languette de verrouillage (215) et l'évidement de verrouillage (216).

6. Dispositif médical jetable selon la revendication 5, dans lequel :
le siège d'extension (25) a un évidement de mise en prise (254) formé de manière annulaire dans une surface interne du siège d'extension (25) entre le trou débouchant (252) et le trou de communication (253) ; et
le corps lumineux (26) est déposé dans l'évidement de mise en prise (254) du siège d'extension (25).

7. Dispositif médical jetable selon l'une quelconque des revendications 1 à 6, dans lequel :
le corps (10) est un spéculum anal ;
la partie d'inspection (11) du corps (10) est un tube d'inspection transparent (111) ; et
la partie de préhension (12) est une poignée (121) et est raccordée au tube d'inspection (111).

8. Dispositif médical jetable selon l'une quelconque des revendications 1 à 6, dans lequel :
le corps (10A, 10B) est un spéculum vaginal ;
la partie d'inspection (11A) du corps (10, 10A, 10B) a :
une mâchoire supérieure (112A, 112B) ayant un segment de languette (114A, 114B) ;
une mâchoire inférieure (113A, 113B) raccordée de manière pivotante à la mâchoire supérieure (112A, 112B) et ayant un segment de languette (114A, 114B) mobile vers ou à distance du segment de languette (114A, 114B) de la mâchoire supérieure (112A, 112B) ; et
une fenêtre (115A, 115B) formée au niveau des extrémités arrière de la mâchoire supérieure (112A, 112B) et de la mâchoire inférieure (113A, 113B) ; et
la partie de préhension (12A) a deux tiges de commande (122A, 123A) respectivement raccordées à et contrôlant les mouvements de la mâchoire supérieure (112A, 112B) et de la mâchoire inférieure (113A, 113B).

9. Dispositif médical jetable selon la revendication 8, dans lequel :
la partie de mise en place (13B) est formée sur une surface interne de l'une parmi la mâchoire supérieure (112B) et la mâchoire inférieure (113B) adjacente à l'extrémité arrière de ladite mâchoire (112B, 113B) ;
l'ouverture avant est déposée dans un espace qui est formé par la mâchoire supérieure (112B) et la mâchoire inférieure (113B) ; et
l'ouverture arrière est déposée entre les mâchoires supérieure et inférieure (112B, 113B) adjacentes à la fenêtre (115B).
